# EUROPEAN PATENT APPLICATION

(11) **EP 4 477 198 A1**
(43) Date of publication of application: **18.12.2024**
(21) Application number: 23178943.9
(22) Date of filing: 13.06.2023
(51) Int. Cl.: A61F 13/15, A61F 13/49, A61F 13/496

(54) **AN APPARATUS AND METHOD OF FORMING TORSO ENCIRCLING PORTIONS FOR PANT-STYLE DIAPER PRODUCTS**

(71) Applicant: Curt G. Joa, Inc., Sheboygan Falls, Wisconsin 53085-0903 (US)
(72) Inventor: SCHWARTZ, Christopher A., Howards Grove, Wisconsin 53083 (US); FRITZ, Jeffrey W., Plymouth, Wisconsin 53073 (US); SCHUETTE, David E., Sheboygan, Wisconsin 53083 (US)
(74) Representative: Potter Clarkson

(57) **Abstract**

Apparatus for manufacturing torso encircling portions for use in the manufacture of pant-style diaper products includes first and second web infeed assemblies to feed a first continuous web and a second continuous web, the first continuous web forming a wider first back sheet strip defining continuous, side-by-side waist and leg sections, and a narrower second back sheet strip defining a continuous waist section, and the second continuous web forming narrower first and second top sheet strips and a wider third sheet strip. A first slitting assembly receives and slits the first continuous web along a first cut line extending in the feed direction to separate the first and second back sheet strips. A second slitting assembly receives and slits the second continuous web along second and third cut lines extending in the feed direction to separate the first, second and third top sheet strips. A waist elastic infeed supplies tensioned waist elastic strands onto the waist sections of the first and second back sheet strips. First and second waist feed assemblies sandwich the waist elastic strands between the waist section of the first back sheet strip and the first top sheet strip and between the waist section of the second back sheet strip and the second top sheet strip. At least one welding assembly welds the waist section of the first back sheet to the first top strip and welds the second back sheet strip to the second top strip intermittently in the feed direction to entrap the waist elastic strands therebetween. A leg elastic infeed inserts tensioned leg elastic in a non-linear pattern onto leg adhesive applied by an adhesive unit and a leg feed assembly overlays the third top sheet strip on the first back sheet strip with the leg elastic strands therebetween.

## Description

The invention relates to a method of manufacturing torso encircling portions for use in the manufacture of pant-style diaper products, and apparatus for manufacturing the torso-encircling portions. More specifically, the invention relates to an apparatus and associated method for manufacturing two-ply torso encircling portions having waist elastic strands secured by means of an adhesive-free welding unit and non-linearly applied leg elastic strands secured by means of adhesive.

Wearable absorbent products including, for example, adult incontinence products, enuresis pants, training pants and other pant-style diapers typically incorporate elasticated torso encircling portions intended to extend around a wearer's waist and keep the article snug against the wearer whilst being worn. Elasticated sections are also preferably provided around the leg openings in order to hold the article snug against a wearer's legs and prevent leakage of urine.

The torso encircling portions may incorporate waist elastic strands, sandwiched under tension between inner and outer layers and arranged to encircle a wearer's waist to provide the required stretch, with leg elastic strands similarly sandwiched under tension between the inner and outer layers around the leg openings. In such arrangements, the elastic strands are typically secured in position between the inner and outer layers of the torso encircling portions by means of adhesive.

The adhesive used in the manufacture of such torso-encircling portions of pant-style diapers is, however, relatively expensive as a result of the cost of the adhesive and also the need for complex delivery systems to apply the adhesive during manufacture. Welding has been used as an alternative to secure waist elastic strands between the inner and outer layers. Welding has not been found to be a reliable alternative to adhesive for securing the leg elastic strands, however, due to processing challenges associated with welding non-linear elastic strands. Thus, a need exists for an apparatus and method for manufacturing a torso-encircling portion of a pant-style diaper that leverages the advantages of welding while mitigating the additional material costs associated with the use of adhesive.

According to an aspect of the invention there is provided an apparatus for manufacturing torso encircling portions for use in the manufacture of pant-style diaper products that includes first and second web infeed assemblies to feed a first continuous web and a second continuous web in a feed direction, the first continuous web forming a wider first back sheet strip defining continuous, side-by-side waist and leg sections extending in the feed direction, and a narrower second back sheet strip defining a continuous waist section extending in the feed direction, and the second continuous web forming narrower first and second top sheet strips and a wider third top sheet strip, and a first slitting assembly positioned to receive and slit the first continuous web along a first cut line extending in the feed direction to separate the first and second back sheet strips. A second slitting assembly is positioned to receive and slit the second continuous web along second and third cut lines extending in the feed direction to separate the first, second and third top sheet strips. A waist elastic infeed is positioned to supply waist elastic strands under tension in the feed direction onto the waist section of the first back sheet strip and onto the waist section of the second back sheet strip. First and second waist feed assemblies are positioned to direct the first and second top sheet strips to sandwich the waist elastic strands between the waist section of the first back sheet strip and the first top sheet strip, and between the waist section of the second back sheet strip and the second top sheet strip. At least one welding assembly welds the waist section of the first back sheet to the first top strip intermittently in the feed direction to entrap the waist elastic strands therebetween, and to weld the second back sheet strip to the second top strip intermittently in the feed direction to entrap the waist elastic strands therebetween. An adhesive unit is positioned to apply leg adhesive to the leg section of the first back sheet strip; a leg elastic infeed is positioned to insert leg elastic strands under tension generally in a feed direction in a non-linear pattern onto the leg adhesive; and a leg feed assembly is positioned to direct the third top sheet strip to overlay the first back sheet strip and sandwich the leg elastic strands therebetween.

Other preferred features of the apparatus are defined in dependent Claims 2-12 and further described below.

According to another aspect of the invention, there is provided a method of manufacturing torso encircling portions for use in the manufacture of pant-style diaper products comprising the steps of operating first and second web infeed assemblies to feed a first continuous web and a second continuous web in a predetermined feed direction, the first continuous web forming a wider first back sheet strip defining continuous, side-by-side waist and leg sections extending in the feed direction, and a narrower second back sheet strip defining a continuous waist section extending in the feed direction, and the second continuous web forming narrower first and second top sheet strips and a wider third top sheet strip; operating a first slitting assembly to slit the first continuous web along a first cut line extending in the feed direction to separate the first and second back sheet strips; and operating a second slitting assembly to slit the second continuous web along second and third cut lines extending in the feed direction to separate the first, second and third top sheet strips. The method also includes operating a waist elastic infeed to feed waist elastic strands under tension between the waist section of the first back sheet strip and the first top sheet strip and between the waist section of the second back sheet strip and the second top sheet strip; operating at least one welding assembly to weld the waist sections of the first back sheet strip and the first top strip intermittently in the feed direction to entrap the waist elastic strands therebetween and to weld the waist section of the second back sheet strip and the second top strip intermittently in the feed direction to entrap the waist elastic strands therebetween; and applying adhesive to leg elastic strands with an adhesive applicator. The method further includes operating a leg elastic infeed to apply the leg elastic strands under tension between the leg section of the first back sheet strip and the third top sheet strip in a non-linear manner relative to the feed direction.

The apparatus and method of the invention results in a two-layer construction across waist and leg sections of torso encircling portions in which waist elastic strands are entrapped by means of welding in waist sections, and non-linearly applied leg elastic strands are secured by means of adhesive in a leg section.

The use of welding in the waist sections provides a clean and simple mechanism when compared with the use of adhesive, and results in a construction that is less sensitive to temperature and softer in feel because the elastic strands are entrapped by means of welds between the top and bottom sheet strips as opposed to being bonded in place by means of adhesive.

The use of welding to entrap elastic strands between top and back sheet strips utilizes a grooved surface to locate the elastic strands relative to one or more welds. As a result, welding is used with strands that are applied in straight lines as opposed to non-linearly applied elastic strands, such as those that are required to form elasticated leg openings.

The apparatus and method advantageously avoids the need to attach separate, elasticated leg portions and instead allows the creation of elasticated leg portions formed integrally with elasticated waist sections whilst avoiding the need for additional layers of web material. As a result, the resultant torso encircling portions are less bulky, allowing for a more discrete fitment.

Minimizing the layers of materials used also has benefits in terms of reducing material cost and the volume of material that must be disposed of in landfill and other waste disposal facilities after articles incorporating the torso encircling portions have been worn.

In embodiments of the invention, the first and second slitting assemblies may be located upstream of the waist elastic infeed.

In other embodiments, the second slitting assembly may be located upstream of the waist elastic infeed whilst the first slitting assembly is located downstream of the at least one welding assembly. In such embodiments, the second continuous web is slit along the second and third cut lines to separate the first, second and third top sheet strips so as to allow positioning of the first, second and third top sheet strips relative to the first and second back sheet strips prior to slitting of the first continuous web along the first cut line to separate the first and second back sheet strips.

Preferably, the waist elastic strands are inserted so as to extend in straight lines along the respective waist sections in the feed direction, spaced from each other in a direction transverse to the feed direction so as to provide a desired stretch effect.

The leg elastic strands are preferably inserted so as to extend in a periodic wave pattern along the leg section in the feed direction so as to allow leg openings to be cut at regular intervals in the torso encircling portions.

So as to provide a neat edge, and improve the comfort of the waist sections when the torso encircling portions are used to form pant-style diapers, an outer edge of each of the first and second back sheet strips may be folded during the step of inserting and sandwiching the waist elastic strands so that the respective top sheet strip overlays the folded edge of each back sheet strip.

In other embodiments, the outer edge of each of the first and second back sheet strips may be folded after the step of inserting and sandwiching the waist elastic strands and before welding so that the folded edge of each back sheet strip overlays an outer edge of the respective top sheet strip.

In yet further embodiments, the outer edge of each of the first and second back sheet strips may be folded after welding and bonded by means of adhesive so as to overlay an outer edge of the respective top sheet strip.

Preferably, so as to improve the comfort and fit of pant-style diapers formed from the resultant torso encircling portions, the method further includes the step of inserting and bonding belly elastic strands extending under tension in the feed direction between the leg section of the first back sheet strip and third top sheet strip by means of adhesive.

In such embodiments, the belly elastic strands are preferably inserted and bonded so as to extend in substantially straight lines along the respective leg sections in the feed direction, equidistantly spaced from each other in a direction transverse to the feed direction so as to provide a unform stretch effect.

So as to avoid the exposure of any adhesive in the resultant torso encircling portions, an inner edge of the third top sheet strip is preferably located so as to overlay, and is bonded to, an inner edge of the first top sheet strip.

The method may include the step of feeding the first and second continuous webs from a single roll of material arranged to deliver a single continuous feeder web in the feed direction that is slit along a fourth cut line extending in the feed direction to form the first and second continuous webs.

In other embodiments, the method may include the step of feeding the first and second continuous webs from a single roll of material arranged to deliver a continuous supply web in the feed direction that is slit along the first, second and third cut lines to form the first and second back sheet strips and the first, second, and third top sheet strips.

In particularly preferred embodiments, the first and second continuous webs are formed from a non-woven thermoplastic material, and the welding steps are performed by means of ultrasonic welding.

Preferably, in such embodiments of the invention, the step of welding the waist sections of the first and second back sheet strips with the respective top sheet strips and waist elastic strands includes feeding the waist elastic strands sandwiched between the respective top and back sheet strips between at least one blade-style horn with a grooved working surface and at least one opposing anvil with a smooth or grooved working surface. The waist elastic strands are fed under tension between the working surface of the horn(s) and the working surface of the anvil(s). The respective top and back sheet strips are then welded to each other via resulting welds that anchor (i.e., entrap) the elastic strands in position relative to the top and back sheet strips intermittently in the feed direction.

In embodiments, one or more rotary or blade-style ultrasonic horns with a smooth or grooved work surface might be used in combination with one or more opposing anvils having a grooved working surface such that the waist elastic strands are fed under tension in the feed direction between the grooves formed in the working surface of the anvil(s) and any grooves in the working surface of the horn(s) so as to allow welding of the respective top and back sheet strips to each other on opposing sides of each of the waist elastic strands intermittently in the feed direction.

Anchoring welds are formed when the working surfaces of the horn(s) and anvil(s) engage sheets located on opposite sides of the elastic strands to melt and at least partially encapsulate the elastic strands and/or frictionally engage the elastic strands in position relative to the facing sheets welds. The resulting anchoring welds may be pairs of welds that are positioned on opposing sides of a given elastic strand and that, in some embodiments, partially overlap the given elastic strand, or as a singular weld or pair of welds that span(s) at least the width of the elastic strand. The anchoring (i.e., entrapment) of the waist elastic strands relative to the facing sheets provides the required elastication in waist sections of the resultant torso encircling portions.

The system may be better understood with reference to the following drawings and the description. The components in the figures are not necessarily to scale, emphasis instead being placed upon illustrating the principles disclosed. Moreover, in the figures, like-referenced numerals designate corresponding parts throughout the different views.

Preferred embodiments of the invention will now be described by way of reference to the accompanying drawings in which:
FIG. 1 shows a pant-style diaper product incorporating elasticated torso encircling portions manufactured in accordance with an embodiment of the invention;
FIG. 2A and 2B show cross-sectional views of the elasticated torso encircling portions of the pant-style diaper product shown in FIG. 1;
FIG. 3 is a schematic illustration of the steps of a method of manufacturing elasticated torso encircling portions in accordance with an embodiment of the invention;
FIG. 4 is a schematic view of an apparatus for manufacturing elasticated torso encircling portions in accordance with an embodiment of the invention;
FIG. 5 is a schematic illustration of the steps of a method of manufacturing elasticated torso encircling portions in accordance with another embodiment of the invention; and
FIG. 6 is a schematic view of an apparatus for manufacturing elasticated torso encircling portions in accordance with another embodiment of the invention.

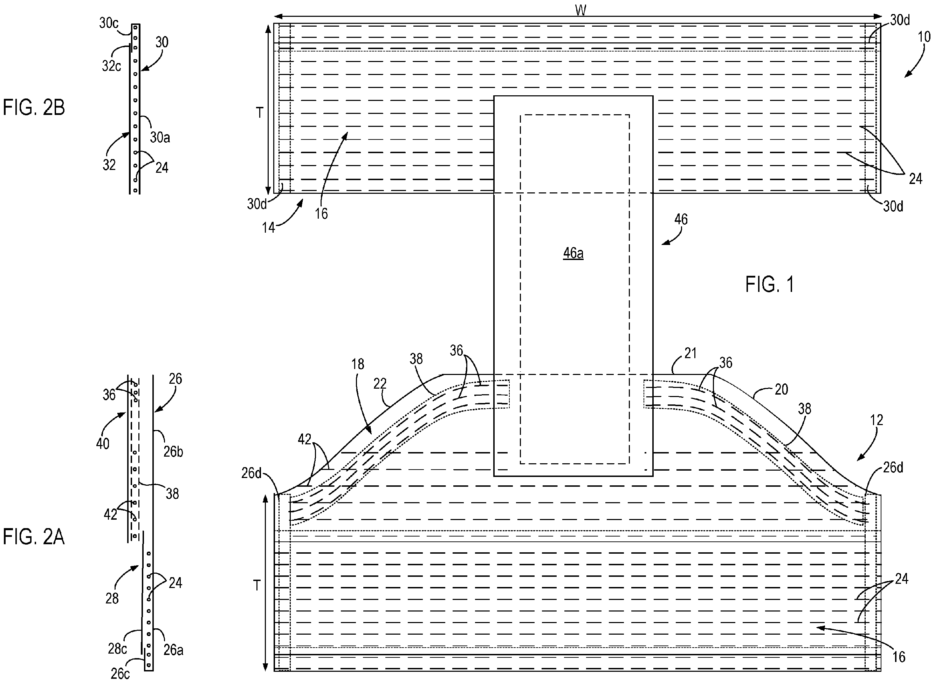

FIGS. 1, 2A, and 2B show a pant-style diaper product 10 incorporating first and second elasticated torso encircling portions 12,14 manufactured in accordance with an embodiment of the invention.

The first and second torso encircling portions 12,14 both define a waist region 16. The first torso encircling portion 12 additionally includes a leg region 18 integrally formed with the respective waist region 16, the leg region 18 having curved edges 20,22 separated by a relatively straight edge 21 so as to extend around a wearer's legs when the diaper product 10 is worn.

Referring to FIG. 2A, it can be seen that the waist region 16 of the first torso encircling portion 12 includes a plurality of waist elastic strands 24 sandwiched between a waist section 26a of a first back sheet strip 26 and a first top sheet strip 28. The waist elastic strands 24 are arranged to extend across all of or substantially all of the width W of the waist region 16. The waist elastic strands 24 may be equidistantly spaced from each other in a transverse direction T relative to the width W or be spaced at varying distances from each other according to design specifications.

The waist section 26a of the first back sheet strip 26 and the first top sheet strip 28 are welded to each other on opposite sides of each of the waist elastic strands at intermittent locations along the length of the waist elastic strands 24 so as to anchor or entrap the waist elastic strands 24 in position relative to the first back sheet strip 26 and the first top sheet strip 28 at those locations.

Similarly, as shown in FIG. 2B, the waist region 16 of the second torso encircling portion 14 includes a plurality of waist elastic strands 24 sandwiched between a waist section 30a of the second back sheet strip 30 and a second top sheet strip 32. As with the waist region 16 of the first torso encircling portion 12, the waist elastic strands 24 are arranged to extend across all of or substantially all of the width W of the waist region 16, and may be equidistantly spaced from each other or spaced at varying distances in a transverse direction T relative to the width W.

The waist section 30a of the second back sheet strip 30 and the second top sheet strip 32 are welded to each other on opposite sides of each of the waist elastic strands at intermittent locations along the length of the waist elastic strands 24 so as to anchor or entrap the waist elastic strands 24 in position relative to the second back sheet strip 30 and the second top sheet strip 32 at those locations.

The leg region 18 of the first torso encircling portion 12 includes a plurality of leg elastic strands 36 sandwiched between a leg section 26b of the first back sheet strip 26 and a third top sheet strip 40, the leg elastic strands 36 being arranged in one exemplary embodiment to follow the outline of the curved edges 20,22 of the leg region 18. The leg elastic strands 36 are bonded in place by means of adhesive 38 provided between the leg section 26b of the first back sheet strip 26 and the third top sheet strip 40.

In the embodiment shown in FIGS. 1, 2A, and 2B, a plurality of belly elastic strands 42 are also sandwiched between the leg section 26b of the first back sheet strip 26 and the third top sheet 40 by means of the adhesive 38. The belly elastic strands 42 are arranged to extend across all of or substantially all of the width W of the leg region 18, and may be equidistantly spaced from each other in a transverse direction T relative to the width W or spaced apart at varying intervals. In other embodiments, the belly elastic strands 42 may be omitted.

So as to provide a smooth finished edge to the waist region 16 of each of the first and second torso encircling portions 12,14, an outer edge 26c of the first back sheet strip 26 is folded over and trapped between an outer edge 28c of the first top sheet strip 28. An outer edge 30c of the second back sheet strip 30 is similarly folded over and trapped between an outer edge 32c of the second top sheet strip 32.

In the embodiment shown in FIGS. 1, 2A, and 2B, the outer edges 26c,30c of the first and second back sheet strips 26,30 are secured in place by means of welding at intermittent locations along the length of elastic waist strands 24 sandwiched between the outer edges 26c,30c and the respective back sheet strips 26,30.

In other embodiments, the outer edges 26c,30c may be folded to overlay the outer edges 28c,32c of the respective top sheet strips 28,32 and secured in position by means of adhesive.

While the product 10 is shown in FIG. 1 in an open format for clarity, it will be understood that the first torso encircling portion 12 and the second the first torso encircling portion 14 are joined together at side seam regions 26d,30d in the completed product 10. Side seam regions 26d,30d may be secured by means of adhesive or ultrasonic welds.

In use, the top sheet strips 28,32,38 define a skin contact surface of the diaper product 10 whilst the back sheet strips 26,30 defines and outer surface of the diaper product 10. The back and top sheet strips 26,28,32,38 are preferably formed from non-woven materials.

Preferably, the third top sheet 32 is arranged to overlay the leg section 26b of the first back sheet strip 26 so that an inner edge 32e of the third top sheet 32 overlays an inner edge 28e of the first top sheet strip 28. This ensures no adhesive is exposed on an outer, skin contacting surface of the resultant torso encircling portion 12.

As shown in FIG. 1, the first and second torso encircling portions 12,14 are bridged by a crotch portion 46 coupled at one end to the leg region 18 of the first torso encircling portion 12 and at its other end to the waist region 16 of the second torso encircling portion 14. The crotch portion 46 includes a liquid absorbent layer on an upper surface 46a and an outer, liquid impermeable layer on its underside (not shown).

The elasticity provided by the waist elastic strands 24 and belly elastic strands 42 may be deactivated (i.e., cut or otherwise treated to reduce the elasticity therein) in these regions so as to prevent bunching of the crotch portion 46.

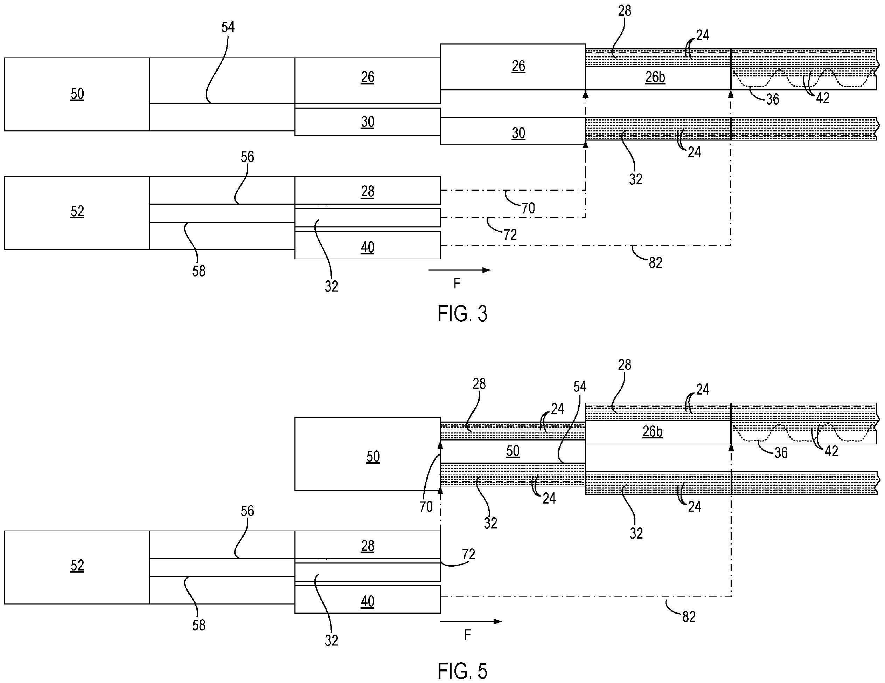

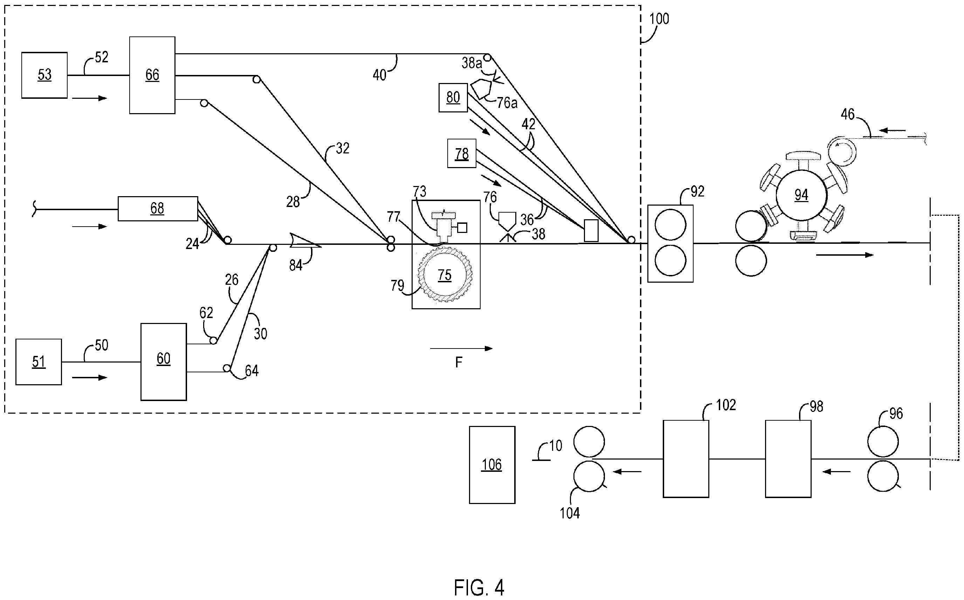

A method and apparatus for forming the first and second torso encircling portions 12,14 will now be described with reference to FIG. 3 and the apparatus 100 shown in FIG. 4.

As a first step, first and second continuous webs 50,52 of nonwoven material are fed in a predetermined machine feed direction F by means of first and second web infeed assemblies 51,53.

The first continuous web 50 is slit by means of a first slitting assembly 60 comprising one or more knives, blades, or other cutting devices, along a first cut line 54 extending in the feed direction F to form the first and second back sheet strips 26,30. The first cut line 54 is located relative to the width of the first continuous web 50 so that the first back sheet strip 26 is wider than the second back sheet 30 and defines continuous, side by side waist and leg sections 26a,26b extending in the feed direction F and a second back sheet strip 30 defining a continuous waist section 30a extending in the feed direction F.

The first and second back sheet strips 26,30 are directed downstream from the slitting assembly 60 by means of one or more web spreaders 62,64 so as to be arranged adjacent to but spaced from each other in a transverse (i.e., cross-machine) direction T relative to the feed direction F and arranged with the leg section 26b of the first back sheet 26 located between the waist sections 26a and 30a.

The second continuous web 52 is slit by means of a second slitting assembly 66 comprising one or more knives, blades, or other cutting devices, along second and third cut lines 56,58 extending in the feed direction F to form first and second top sheet strips 28,32 and a third top sheet strip 40. The second and third cut lines 56,58 are located relative to the width of the second continuous 52 so that the third top sheet strip 40 is wider than the first and second top sheet strips 28,32.

A waist elastic infeed in the form of a harp 68 inserts waist elastic strands 24 extending in the feed direction F under tension so as to overlay the waist section 26a of the first back sheet strip 26 and the waist section 30a of the second back sheet strip 30. The harp 68 feeds the waist elastic strands 24 in substantially straight lines in the feed direction F along the waist sections 26a,30a of the first and second back sheet strips 26,30. The waist elastic strands 24 may be equidistantly spaced in a direction T transverse to the feed direction F or be fed at spacing that varies in the transverse direction T between waist elastic strands 24 according to product design specifications.

The first and second back sheet strips 26,30 then pass through one or more waist edge folding units 84 to fold outer edges 26c,30c of the waist sections 26a,30a of the first and second back sheet strips 26,30 so as to overlay the outermost waist elastic strands 24.

The first top sheet strip 28 is then directed via a first waist feed assembly 70 to overlay the waist section 26a of the first back sheet strip 26 so as to sandwich waist elastic strands 24 therebetween, and the second top sheet strip 32 is directed via a second waist feed assembly 72 to overlay the waist section 30a of the second back sheet strip 30 so as to sandwich waist elastic strands 24 therebetween. The first and second top sheet strips 28,32 overlay the outer edges 26c,30c.

In order to secure the waist sections of the respective top and back sheets together and retain the folded outer edges 26c,30c and waist elastic strands 24 in position, the waist sections of the respective top and back sheets and waist elastic strands 24 sandwiched therebetween are fed through at least one welding assembly 74.

The at least one welding assembly 74 may be any known mechanical bonding system including, as non-limiting examples, a thermal welding system, a pressure welding system, a rotary ultrasonic welding system, or a blade ultrasonic welding system, any of which include one or more components that cooperate to weld (i.e., fuse) the top and back sheets together. In one embodiment, the welding assembly 74 is provided in the form of an ultrasonic bonding system having one or more blade-style horns 73, also known as a sonotrode, with a grooved working surface 77 that cooperate with one or more opposing anvils 75 with smooth or grooved ridges 79. In other embodiments, the welding assembly 74 may be provided in the form of an ultrasonic bonding system including one or more rotary or blade-style horn(s) having either a smooth or grooved working surface and one or more anvils with grooved ridges. While welding assembly 74 and corresponding horn and anvil elements 73 and 75 are referred to in the singular tense below, it is contemplated that any of the described embodiments may include multiple welding assemblies, each including one or more horn and/or anvil components.

The waist sections of the respective top and back sheets and waist elastic strands 24 are fed through the welding assembly 74 so that the waist elastic strands 24 under tension in the feed direction F are aligned with the grooves formed in the working surface of the ultrasonic horn. Intermittent operation of the ultrasonic horn 73 forms welds 34 at intermittent locations along the length of the waist elastic strands 24 as the waist sections of the respective top and back sheets and waist elastic strands 24 are fed through the welding assembly 74.

Operation of the ultrasonic horn 73 melts the nonwoven material of the respective top and back sheet strips at the points of contact between the horn 73 and the anvil 75, which results in the formation of welds 34 between the respective top and back sheet strips, the welds 34 being aligned with land surfaces between the grooves in the working surface of the ultrasonic horn 73 so that they entrap the waist elastic strands 24.

Following welding of the waist sections to entrap the waist elastic strands 24 and form waist regions 16, the leg section 26b of the first back sheet strip 26 passes an adhesive unit 76 that applies adhesive 38 to the exposed surface of the leg section 26b of the first back sheet strip 26. Optionally, apparatus 100 may include one or more web guides and/or spreaders (not shown) located downstream from the welding assembly 74 to reposition the welded top and back sheet webs for later downstream processing after they exit the welding assembly 74.

A leg elastic infeed in the form of a curved leg elastic laydown unit 78 inserts leg elastic strands 36 extending under tension generally in the feed direction F and apply the leg elastic strands 36 to the adhesive 38 in a periodic wave pattern or other non-linear pattern along the leg section 26b in the feed direction F. It will be appreciated that the curved leg elastic laydown unit 78 may be controlled so as to vary the shape defined by the leg elastic strands 36 on the leg section 26b of the first back sheet strip 26 depending on the intended end use of the torso encircling portions 12,14.

A belly elastic infeed 80 may be located downstream (as shown) or upstream of the curved leg elastic laydown unit 78 to insert belly elastic strands 42 extending under tension in the feed direction F on to the adhesive 38 on the leg section 26b of the first back sheet strip 26. The belly elastic infeed 80 feeds the belly elastic strands 42 in substantially straight lines in the feed direction F along the leg section 26b of the first back sheet 26, equidistantly spaced in a direction T transverse to the feed direction F. In other embodiments, the belly elastic strands 42 and associated belly elastic infeed 80 may be omitted.

The third top sheet strip 40 is then directed via a leg feed assembly 82 to overlay the leg section 26b of the first back sheet strip 26 so as to sandwich leg elastic strands 24 and the belly elastic strands 42 therebetween, the third top sheet strip 40 being bonded to the leg section 26b of the first back sheet strip 26 by means of the adhesive 38. Optionally, a second adhesive unit 76a is provided to apply adhesive 38a to the third top sheet strip 40.

Preferably, the third top sheet 32 is arranged to overlay the leg section 26b of the first back sheet strip 26 so that an inner edge 32e of the third top sheet 32 overlays an inner edge 28e of the first top sheet strip 28. This ensures no adhesive is exposed on an outer, skin contacting surface of the resultant torso encircling portion 12. It will be appreciated that the position of the waist edge folding unit 84 could be varied.

The waist edge folding unit 84 could, in other embodiments, be located downstream from the first and second waist feed assemblies 70,72 so that the folded outer edges 26c,30c of the first and second back sheet strips 26,30 overlay the respective top sheet strips 28,32 before passing through the welding assembly 74. In yet further embodiments, the waist edge folding 84 could be located downstream from the welding assembly 74 so that the folded outer edges 26c,30c of the first and second back sheet strips 26,30 overlay the respective top sheet strips 28,32 and adhesive is applied to bond them in position.

The first and second web infeed assemblies 51,53 and the first and second slitting assemblies 60,66 are shown in FIG. 4 arranged in line with the waist elastic infeed. In other embodiments, the first and second web infeed assemblies 51,53 and the first and second slitting assemblies 60,66 may be arranged offline relative to the waist elastic infeed.

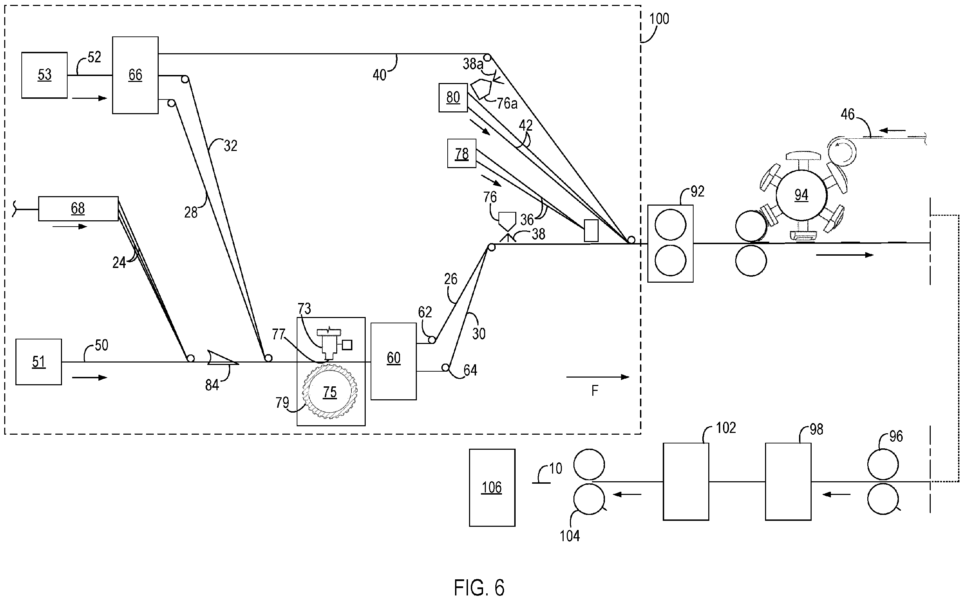

An alternative method for forming the first and second torso encircling portions 12,14 will now be described with reference to FIG. 5 and the apparatus 100 shown in FIG. 6.

As a first step, first and second continuous webs 50,52 of nonwoven material are fed in a predetermined machine feed direction F by means of first and second web infeed assemblies 51,53.

A waist elastic infeed in the form of a harp 68 inserts waist elastic strands 24 extending in the feed direction F under tension so as to overlay waist sections 26a, 30a of the first continuous web 50. The harp 68 feeds the waist elastic strands 24 in substantially straight lines in the feed direction F along the waist sections 26a,30a. The waist elastic strands 24 may be equidistantly spaced in a direction T transverse to the feed direction F or be fed at spacing that varies in the transverse direction T between waist elastic strands 24 according to product design specifications.

The first continuous web 50 then passes through one or more waist edge folding units 84 to fold outer edges 26c,30c of the waist sections 26a,30a of the first continuous web 50 so as to overlay the outermost waist elastic strands 24.

The second continuous web 52 is slit by means of a second slitting assembly 66 comprising one or more knives, blades, or other cutting devices, along second and third cut lines 56,58 extending in the feed direction F to form first and second top sheet strips 28,32 and a third top sheet strip 40. The second and third cut lines 56,58 are located relative to the width of the second continuous 52 so that the third top sheet strip 40 is wider than the first and second top sheet strips 28,32.

The first top sheet strip 28 is then directed via a first waist feed assembly 70 to overlay the waist section 26a of the first continuous web 50 so as to sandwich waist elastic strands 24 therebetween, and the second top sheet strip 32 is directed via a second waist feed assembly 72 to overlay the waist section 30a of the first continuous web 50 so as to sandwich waist elastic strands 24 therebetween. The first and second top sheet strips 28,32 overlay the outer edges 26c,30c.

In order to secure the waist sections of the respective top and back sheets together and retain the folded outer edges 26c,30c and waist elastic strands 24 in position, the waist sections of the respective top and back sheets and waist elastic strands 24 sandwiched therebetween are fed through at least one welding assembly 74.

The at least one welding assembly 74 may be any known mechanical bonding system including, as non-limiting examples, a thermal welding system, a pressure welding system, a rotary ultrasonic welding system, or a blade ultrasonic welding system, any of which include one or more components that cooperate to weld (i.e., fuse) the top and back sheets together. In one embodiment, the welding assembly 74 is provided in the form of an ultrasonic bonding system having one or more blade-style horns 73, also known as a sonotrode, with a grooved working surface 77 that cooperate with one or more opposing anvils 75 with smooth or grooved ridges 79. In other embodiments, the welding assembly 74 may be provided in the form of an ultrasonic bonding system including one or more rotary or blade-style horn(s) having either a smooth or grooved working surface and one or more anvilswith grooved ridges. While welding assembly 74 and corresponding horn and anvil elements 73 and 75 are referred to in the singular tense below, it is contemplated that any of the described embodiments may include multiple welding assemblies, each including one or more horn and/or anvil components.

The waist sections of the respective top and back sheets and waist elastic strands 24 are fed through the welding assembly 74 so that the waist elastic strands 24 under tension in the feed direction F are aligned with the grooves formed in the working surface of the ultrasonic horn. Intermittent operation of the ultrasonic horn 73 forms welds 34 at intermittent locations along the length of the waist elastic strands 24 as the waist sections of the respective top and back sheets and waist elastic strands 24 are fed through the welding assembly 74.

Operation of the ultrasonic horn 73 melts the nonwoven material of the respective top and back sheet strips at the points of contact between the horn 73 and the anvil 75, which results in the formation of welds 34 between the respective top and back sheet strips, the welds 34 being aligned with land surfaces between the grooves in the working surface of the ultrasonic horn 73 so that they entrap the waist elastic strands 24.

Following welding of the waist sections to entrap the waist elastic strands 24 and form waist regions 16, the first continuous web 50 is slit by means of a first slitting assembly 60 comprising one or more knives, blades, or other cutting devices, along a first cut line 54 extending in the feed direction F to form the first and second back sheet strips 26,30. The first cut line 54 is located relative to the width of the first continuous web 50 so that the first back sheet strip 26 is wider than the second back sheet 30 and defines continuous, side by side waist and leg sections 26a,26b extending in the feed direction F and a second back sheet strip 30 defining a continuous waist section 30a extending in the feed direction F. One or more web guides and/or spreaders 62,24 are located downstream from the welding assembly 74 to reposition the welded top and back sheet webs in a transvers (i.e., cross-machine) direction T relative to the feed direction F for later downstream processing after they exit the welding assembly 74. In an alternative embodiment, first slitting assembly 60 is located upstream of welding assembly 74.

The leg section 26b of the first back sheet strip 26 then passes an adhesive unit 76 that applies adhesive 38 to the exposed surface of the leg section 26b of the first back sheet strip 26.

A leg elastic infeed in the form of a curved leg elastic laydown unit 78 inserts leg elastic strands 36 extending under tension generally in the feed direction F and apply the leg elastic strands 36 to the adhesive 38 in a periodic wave pattern or other non-linear pattern along the leg section 26b in the feed direction F. It will be appreciated that the curved leg elastic laydown unit 78 may be controlled so as to vary the shape defined by the leg elastic strands 36 on the leg section 26b of the first back sheet strip 26 depending on the intended end use of the torso encircling portions 12,14.

A belly elastic infeed 80 may be located downstream (as shown) or upstream of the curved leg elastic laydown unit 78 to insert belly elastic strands 42 extending under tension in the feed direction F on to the adhesive 38 on the leg section 26b of the first back sheet strip 26. The belly elastic infeed 80 feeds the belly elastic strands 42 in substantially straight lines in the feed direction F along the leg section 26b of the first back sheet 26, equidistantly spaced in a direction T transverse to the feed direction F. In other embodiments, the belly elastic strands 42 and associated belly elastic infeed 80 may be omitted.

The third top sheet strip 40 is then directed via a leg feed assembly 82 to overlay the leg section 26b of the first back sheet strip 26 so as to sandwich leg elastic strands 24 and the belly elastic strands 42 therebetween, the third top sheet strip 40 being bonded to the leg section 26b of the first back sheet strip 26 by means of the adhesive 38. Optionally, a second adhesive unit 76a is provided to apply adhesive 38a to the third top sheet strip 40.

Preferably, the third top sheet 32 is arranged to overlay the leg section 26b of the first back sheet strip 26 so that an inner edge 32e of the third top sheet 32 overlays an inner edge 28e of the first top sheet strip 28. This ensures no adhesive is exposed on an outer, skin contacting surface of the resultant torso encircling portion 12. It will be appreciated that the position of the waist edge folding unit 84 could be varied.

The waist edge folding unit 84 could, in other embodiments, be located downstream from the first and second waist feed assemblies 70,72 so that the folded outer edges 26c,30c of the first and second back sheet strips 26,30 overlay the respective top sheet strips 28,32 before passing through the welding assembly 74. In yet further embodiments, the waist edge folding 84 could be located downstream from the welding assembly 74 so that the folded outer edges 26c,30c of the first and second back sheet strips 26,30 overlay the respective top sheet strips 28,32 and adhesive is applied to bond them in position.

The first and second web infeed assemblies 51,53 and the first and second slitting assemblies 60,66 are shown in FIG. 6 arranged in line with the waist elastic infeed. In other embodiments, the first and second web infeed assemblies 51,53 and the first and second slitting assemblies 60,66 may be arranged offline relative to the waist elastic infeed.

It is also envisaged that in other embodiments a supply web infeed assembly (not shown) might be located upstream from the first and second web infeed assemblies 51,53 to deliver a single continuous feeder web in the feed direction. In such embodiments, a third slitting assembly could be arranged between the supply web infeed assembly and the first and second web infeed assemblies 51,53 to slit the feeder web along a fourth cut line extending in the feed direction F to provide the first and second continuous webs 50,52 for delivery via the first and second web infeed assemblies 51,53.

It is also envisaged that in other embodiments a supply web infeed assembly (not shown) might be located upstream from the first and second web infeed assemblies 51,53 to deliver a single continuous feeder web in the feed direction.

In yet further embodiments, the first and second web infeed assemblies 51,53 could be provided in the form of a single supply web infeed assembly feeding the first and second continuous webs 50,52 in the feed direction in the form of a single feed web that is slit by the first and second slitting assemblies 60,66 or a single slitting assembly (not shown) that replaces the first and second slitting assemblies 60,66, along the first, second and third cut lines 54,56,58 extending in the feed direction F, to form the first and second back sheet strips 26,30 and the first, second and third top sheet strips 28,32,40.

In yet further embodiments, any of first and second web infeed assemblies 51,53 and one or more additional web infeed assemblies (not shown) may feed web originally sized at the width of first back sheet strip 26, second back sheet strip 30, first top sheet strip 28, second top sheet strip 32, and/or third top sheet strip 40 such that first and/or second slitting assemblies might be omitted.

Further processing of the intermediate torso encircling portions of a pant-style diaper product may occur downstream of apparatus 100 of FIG. 4 or FIG. 6 using equipment and processes known in the art. While the specifics of such additional equipment is outside the scope of the present invention, one skilled in the art will recognize that such equipment may include any combination of all or some of the following exemplary processing units, along with additional processing units as known in the art: a deactivation unit 92, a transfer unit 94 (optionally with cutting functionality) to handle absorbent inserts 46, a cutting unit 96 in which portions of waistbands are cut to create curved leg profile, a folding unit 98 in which one of the first and second torso encircling portion 12,14 is folded over the other, one or more adhesive or ultrasonic bonding units 102 in which the first and second torso encircling portion 12,14 are joined together at side seam regions 26d,30d, a cutting or knife unit 104 in which the continuous web of intermediate pant product is separated into discrete products 10, and/or a transfer unit 106 in which discrete products 10 are picked up and turned into a packaging orientation, and may further represent a packaging folding unit in which the discrete products 10 are folded for packaging.

Embodiments of the invention will now be described in the following numbered paragraphs:
1. Apparatus for manufacturing torso encircling portions for use in the manufacture of pant-style diaper products comprising:
   first and second web infeed assemblies (51,53) to feed a first continuous web and a second continuous web (50,52) in a feed direction (F), the first continuous web forming a wider first back sheet strip defining continuous, side-by-side waist and leg sections extending in the feed direction, and a narrower second back sheet strip defining a continuous waist section extending in the feed direction, and the second continuous web forming narrower first and second top sheet strips and a wider third top sheet strip;
   a first slitting assembly (60) positioned to receive and slit the first continuous web along a first cut line extending in the feed direction to separate the first and second back sheet strips;
   a second slitting assembly (66) positioned to receive and slit the second continuous web along second and third cut lines extending in the feed direction to separate the first, second and third top sheet strips;
   a waist elastic infeed (68) positioned to supply waist elastic strands under tension in the feed direction onto the waist section of the first back sheet strip and onto the waist section of the second back sheet strip;
   first and second waist feed assemblies (70,72) positioned to direct the first and second top sheet strips to sandwich the waist elastic strands between the waist section of the first back sheet strip and the first top sheet strip, and between the waist section of the second back sheet strip and the second top sheet strip;
   at least one welding assembly (74) to weld the waist section of the first back sheet to the first top strip intermittently in the feed direction to entrap the waist elastic strands therebetween, and to weld the second back sheet strip to the second top strip intermittently in the feed direction to entrap the waist elastic strands therebetween;
   an adhesive unit (76) positioned to apply leg adhesive to the leg section of the first back sheet strip;
   a leg elastic infeed (78) positioned to insert leg elastic strands under tension generally in a feed direction in a non-linear pattern onto the leg adhesive; and
   a leg feed assembly (82) positioned to direct the third top sheet strip to overlay the first back sheet strip and sandwich the leg elastic strands therebetween.
2. The apparatus of paragraph 1 wherein the first and second slitting assemblies (60,66) are both located upstream of the waist elastic infeed (68).
3. The apparatus of paragraph 1 wherein second slitting assembly is located upstream of the waist elastic infeed (68) and the first slitting assembly (60) is located downstream of the at least one welding assembly (74).
4. The apparatus of any one of paragraphs 1 to 3 wherein the leg elastic infeed includes one or more reciprocating leg elastic feed elements to insert the leg elastic strands so as to extend in a periodic wave pattern along the leg section in the feed direction.
5. The apparatus of any one of paragraphs 1 to 4 further including one or more waist edge folding units (84) located downstream from the waist elastic infeed assembly upstream of the second waist feed assembly to fold an outer edge of each of the first and second back sheet strips so that the respective top sheet strip overlays the folded edge of each back sheet strip prior to travel in the feed direction through the welding assembly.
6. The apparatus of any one of paragraphs 1 to 4 further including one or more waist edge folding units (84) located downstream from the waist feed assembly and the second waist feed assembly to fold an outer edge of each of the first and second back sheet strips so that the folded edge of each back sheet strip overlays an outer edge of the respective top sheet strip.
7. The apparatus of any one of paragraphs 1 to 6 further including a belly elastic infeed (80) located upstream from the leg feed assembly in the feed direction and positioned to insert belly elastic strands extending under tension in the feed direction onto the adhesive on the leg section of the first back sheet strip, wherein the leg feed assembly directs the third top sheet strip to overlay the leg section of the first back sheet strip so as to sandwich leg elastic strands and the belly elastic strands therebetween.
8. The apparatus of any one of paragraphs 1 to 6 further including one or more web spreaders (62,64) to arrange the first and second back sheet strips adjacent to but spaced from each other in a direction transverse to the feed direction and arranged with the leg section of the first back sheet strip located between the waist sections.
9. The apparatus of any one of paragraphs 1 to 8 wherein the welding assembly comprises at least one blade-style ultrasonic horn with a grooved working surface and at least one opposing anvil with smooth or grooved ridges, the horn(s) and anvil(s) being arranged on opposing sides of a feed path through the welding assembly so that the waist elastic strands sandwiched between the waist section of the first back sheet and first top sheet, and sandwiched between the waist section of second back sheet and second top sheet, travel in the feed direction between the horn(s) and anvil(s).
10. The apparatus of any one of paragraphs 1 to 8 wherein the welding assembly includes at least one rotary or blade-style ultrasonic horn with a smooth or grooved working surface and at least one opposing anvil with grooved ridges, the horn(s) and anvil(s) being arranged on opposing sides of a feed path through the welding assembly so that the waist elastic strands sandwiched between the waist section of the first back sheet and first top sheet, and sandwiched between the waist section of second back sheet and second top sheet, travel in the feed direction between the horn(s) and anvil(s).
11. The apparatus of any one of paragraphs 1 to 10 wherein the first and second web infeed assemblies and the first and second slitting assemblies are arranged in line with the waist elastic infeed.
12. The apparatus of any one of paragraphs 1 to 10 wherein the first and second web infeed assemblies and the first and second slitting assemblies are arranged offline relative to the waist elastic infeed.
13. The apparatus of any one of paragraphs 1 to 12 further including a supply web infeed assembly arranged to deliver a single continuous feeder web in the feed direction and a third slitting assembly arranged to slit the feeder web along a fourth cut line extending in the feed direction to form the first and second continuous webs.
14. The apparatus of any one of paragraphs 1 to 12 wherein the first and second web infeed assemblies are provided in the form of a single supply web infeed assembly feeding the first and second continuous webs in the feed direction in the form of a single feed web that is slit by the first and second slitting assemblies, along the first, second and third cut lines extending in the feed direction, to form the first and second back sheet strips and the first, second and third top sheet strips.
15. A method of manufacturing torso encircling portions for use in the manufacture of pant-style diaper products comprising the steps of:
   operating first and second web infeed assemblies (51,53) to feed a first continuous web and a second continuous web in a predetermined feed direction, the first continuous web forming a wider first back sheet strip defining continuous, side-by-side waist and leg sections extending in the feed direction, and a narrower second back sheet strip defining a continuous waist section extending in the feed direction, and the second continuous web forming narrower first and second top sheet strips and a wider third top sheet strip;
   operating a first slitting assembly (60) to slit the first continuous web along a first cut line extending in the feed direction to separate the first and second back sheet strips;
   operating a second slitting assembly (66) to slit the second continuous web along second and third cut lines extending in the feed direction to separate the first, second and third top sheet strips;
   operating a waist elastic infeed (68) to feed waist elastic strands under tension between the waist section of the first back sheet strip and the first top sheet strip and between the waist section of the second back sheet strip and the second top sheet strip;
   operating at least one welding assembly (74) to weld the waist sections of the first back sheet strip and the first top strip intermittently in the feed direction to entrap the waist elastic strands therebetween and to weld the waist section of the second back sheet strip and the second top strip intermittently in the feed direction to entrap the waist elastic strands therebetween;
   applying adhesive to leg elastic strands with an adhesive applicator (76); and
   operating a leg elastic infeed (78) to apply the leg elastic strands under tension between the leg section of the first back sheet strip and the third top sheet strip in a non-linear manner relative to the feed direction.
16. The method of paragraph 15 wherein the steps of operating the first and second slitting assemblies (60,66) to slit the first and second continuous webs is performed upstream of the at least one welding assembly (74).
17. The method of paragraph 15 wherein the step of operating the second slitting assembly (66) to slit the second continuous web is performed upstream of the at least one welding assembly (74) and the step of operating the first slitting assembly (60) to slit the first continuous web is performed downstream of the at least one welding assembly (74).
18. The method of any one of paragraphs 15 to 17 further comprising operating the leg elastic infeed to apply the leg elastic strands in a periodic wave pattern along the leg section in the feed direction.
19. The method of any one of paragraphs 15 to 18 wherein the step of welding the waist sections of the first and second back sheet strips with the respective top sheet strips and waist elastic strands includes feeding the waist elastic strands sandwiched between the respective top and back sheet strips between at least one blade-style ultrasonic horn with a grooved working surface and at least one opposing anvil with smooth or grooved ridges, and welding the respective top and back sheet strips to each other intermittently in the feed direction so as to entrap the waist elastic strands therebetween.
20. The method of any one of paragraphs 15 to 18 wherein the step of welding the waist sections of the first and second back sheet strips with the respective top sheet strips and waist elastic strands includes feeding the waist elastic strands sandwiched between the respective top and back sheet strips between at least one rotary horn or at least one blade-style ultrasonic horn with a smooth or grooved working surface and at least one opposing anvil with grooved ridges, and welding the respective top and back sheet strips to each other intermittently in the feed direction so as to entrap the waist elastic strands therebetween.
21. The method of any one of paragraphs 15 to 20 further comprising operating a folding unit (84) to fold an outer edge of each of the first and second back sheet strips and overlaying the respective top sheet strip on the folded edge of each back sheet strip.
22. The method of any one of paragraphs 15 to 20 further comprising operating at least one folding unit (84) to fold an outer edge of each of the first and second back sheet strips to overlay an outer edge of the respective top sheet strip.
23. The method of any one of paragraphs 15 to 22 further including operating a belly elastic infeed (80) to insert belly elastic strands under tension in the feed direction between the leg section of the first back sheet strip and the third top sheet strip; and
   securing the belly elastic strands to the first back sheet strip by means of an adhesive applicator (76).
24. The method of any one of paragraphs 15 to 23 further including operating at least one spreading unit (62,64) to space the first and second back sheet strips from each other in a direction transverse to the feed direction and to locate the leg section of the first back sheet strip between the waist section of the first back sheet strip and the waist section of the second back sheet strip.
25. The method of any one of paragraphs 15 to 24 further including the step of feeding the first and second continuous webs from a single roll of material arranged to deliver a single feed web in the feed direction and operating the at least one slitting unit and the at least one spreading unit to slit along a fourth cut line extending in the feed direction to form the first and second continuous webs.
26. The method of any one of paragraphs 15 to 24 further including the step of feeding the first and second continuous webs from a single roll of material arranged to deliver a web in the feed direction and operating the at least one slitting unit and the at least one spreading unit to slit along the first, second and third cut lines to form the first and second back sheet strips and the first, second, and third top sheet strips.

## Claims

1. Apparatus for manufacturing torso encircling portions for use in the manufacture of pant-style diaper products comprising:
first and second web infeed assemblies (51,53) to feed a first continuous web and a second continuous web (50,52) in a feed direction (F), the first continuous web forming a wider first back sheet strip defining continuous, side-by-side waist and leg sections extending in the feed direction, and a narrower second back sheet strip defining a continuous waist section extending in the feed direction, and the second continuous web forming narrower first and second top sheet strips and a wider third top sheet strip;
a first slitting assembly (60) positioned to receive and slit the first continuous web along a first cut line extending in the feed direction to separate the first and second back sheet strips;
a second slitting assembly (66) positioned to receive and slit the second continuous web along second and third cut lines extending in the feed direction to separate the first, second and third top sheet strips;
a waist elastic infeed (68) positioned to supply waist elastic strands under tension in the feed direction onto the waist section of the first back sheet strip and onto the waist section of the second back sheet strip;
first and second waist feed assemblies (70,72) positioned to direct the first and second top sheet strips to sandwich the waist elastic strands between the waist section of the first back sheet strip and the first top sheet strip, and between the waist section of the second back sheet strip and the second top sheet strip;
at least one welding assembly (74) to weld the waist section of the first back sheet to the first top strip intermittently in the feed direction to entrap the waist elastic strands therebetween, and to weld the second back sheet strip to the second top strip intermittently in the feed direction to entrap the waist elastic strands therebetween;
an adhesive unit (76) positioned to apply leg adhesive to the leg section of the first back sheet strip;
a leg elastic infeed (78) positioned to insert leg elastic strands under tension generally in a feed direction in a non-linear pattern onto the leg adhesive; and
a leg feed assembly (82) positioned to direct the third top sheet strip to overlay the first back sheet strip and sandwich the leg elastic strands therebetween.

2. Apparatus according to Claim 1 wherein the first and second slitting assemblies (60,66) are both located upstream of the waist elastic infeed (68) or wherein second slitting assembly is located upstream of the waist elastic infeed (68) and the first slitting assembly (60) is located downstream of the at least one welding assembly (74).

3. Apparatus according to Claim 1 or Claim 2 wherein the leg elastic infeed includes one or more reciprocating leg elastic feed elements to insert the leg elastic strands so as to extend in a periodic wave pattern along the leg section in the feed direction.

4. Apparatus according to any one of Claims 1 to 3 further including one or more waist edge folding units (84) located downstream from the waist elastic infeed assembly and either upstream of the second waist feed assembly to fold an outer edge of each of the first and second back sheet strips so that the respective top sheet strip overlays the folded edge of each back sheet strip prior to travel in the feed direction through the welding assembly or downstream from the second waist feed assembly to fold an outer edge of each of the first and second back sheet strips so that the folded edge of each back sheet strip overlays an outer edge of the respective top sheet strip.

5. Apparatus according to any one of Claims 1 to 4 further including a belly elastic infeed (80) located upstream from the leg feed assembly in the feed direction and positioned to insert belly elastic strands extending under tension in the feed direction onto the adhesive on the leg section of the first back sheet strip, wherein the leg feed assembly directs the third top sheet strip to overlay the leg section of the first back sheet strip so as to sandwich leg elastic strands and the belly elastic strands therebetween.

6. Apparatus according to any one of Claims 1 to 5 further including one or more web spreaders (62,64) to arrange the first and second back sheet strips adjacent to but spaced from each other in a direction transverse to the feed direction and arranged with the leg section of the first back sheet strip located between the waist sections.

7. Apparatus according to any one of Claims 1 to 6 wherein the welding assembly comprises:
at least one blade-style ultrasonic horn with a grooved working surface and at least one opposing anvil with smooth or grooved ridges, the horn(s) and anvil(s) being arranged on opposing sides of a feed path through the welding assembly so that the waist elastic strands sandwiched between the waist section of the first back sheet and first top sheet, and sandwiched between the waist section of second back sheet and second top sheet, travel in the feed direction between the horn(s) and anvil(s); or
at least one rotary or blade-style ultrasonic horn with a smooth or grooved working surface and at least one opposing anvil with grooved ridges, the horn(s) and anvil(s) being arranged on opposing sides of a feed path through the welding assembly so that the waist elastic strands sandwiched between the waist section of the first back sheet and first top sheet, and sandwiched between the waist section of second back sheet and second top sheet, travel in the feed direction between the horn(s) and anvil(s).

8. Apparatus according to any one of Claims 1 to 7 wherein the first and second web infeed assemblies and the first and second slitting assemblies are arranged in line with or offline relative to the waist elastic infeed

9. Apparatus according to any one of Claims 1 to 8 further including a supply web infeed assembly arranged to deliver a single continuous feeder web in the feed direction and a third slitting assembly arranged to slit the feeder web along a fourth cut line extending in the feed direction to form the first and second continuous webs; or
wherein the first and second web infeed assemblies are provided in the form of a single supply web infeed assembly feeding the first and second continuous webs in the feed direction in the form of a single feed web that is slit by the first and second slitting assemblies, along the first, second and third cut lines extending in the feed direction, to form the first and second back sheet strips and the first, second and third top sheet strips.

10. A method of manufacturing torso encircling portions for use in the manufacture of pant-style diaper products comprising the steps of:
operating first and second web infeed assemblies (51,53) to feed a first continuous web and a second continuous web in a predetermined feed direction, the first continuous web forming a wider first back sheet strip defining continuous, side-by-side waist and leg sections extending in the feed direction, and a narrower second back sheet strip defining a continuous waist section extending in the feed direction, and the second continuous web forming narrower first and second top sheet strips and a wider third top sheet strip;
operating a first slitting assembly (60) to slit the first continuous web along a first cut line extending in the feed direction to separate the first and second back sheet strips;
operating a second slitting assembly (66) to slit the second continuous web along second and third cut lines extending in the feed direction to separate the first, second and third top sheet strips;
operating a waist elastic infeed (68) to feed waist elastic strands under tension between the waist section of the first back sheet strip and the first top sheet strip and between the waist section of the second back sheet strip and the second top sheet strip;
operating at least one welding assembly (74) to weld the waist sections of the first back sheet strip and the first top strip intermittently in the feed direction to entrap the waist elastic strands therebetween and to weld the waist section of the second back sheet strip and the second top strip intermittently in the feed direction to entrap the waist elastic strands therebetween;
applying adhesive to leg elastic strands with an adhesive applicator (76); and
operating a leg elastic infeed (78) to apply the leg elastic strands under tension between the leg section of the first back sheet strip and the third top sheet strip in a non-linear manner relative to the feed direction.

11. A method according to Claim 10 wherein the steps of operating the first and second slitting assemblies (60,66) to slit the first and second continuous webs is performed upstream of the at least one welding assembly (74) or wherein the step of operating the second slitting assembly (66) to slit the second continuous web is performed upstream of the at least one welding assembly (74) and the step of operating the first slitting assembly (60) to slit the first continuous web is performed downstream of the at least one welding assembly (74).

12. A method according to Claim 10 or Claim 11 further comprising operating the leg elastic infeed to apply the leg elastic strands in a periodic wave pattern along the leg section in the feed direction.

13. A method according to any one of Claims 10 to 12 wherein the step of welding the waist sections of the first and second back sheet strips with the respective top sheet strips and waist elastic strands includes feeding the waist elastic strands sandwiched between the respective top and back sheet strips between (A) at least one blade-style ultrasonic horn with a grooved working surface and at least one opposing anvil with smooth or grooved ridges, and welding the respective top and back sheet strips to each other intermittently in the feed direction so as to entrap the waist elastic strands therebetween or (B) at least one rotary horn or at least one blade-style ultrasonic horn with a smooth or grooved working surface and at least one opposing anvil with grooved ridges, and welding the respective top and back sheet strips to each other intermittently in the feed direction so as to entrap the waist elastic strands therebetween.

14. A method according to any one of Claims 10 to 13 further comprising operating at least one folding unit (84) to fold an outer edge of each of the first and second back sheet strips and overlaying the respective top sheet strip on the folded edge of each back sheet strip or to fold an outer edge of each of the first and second back sheet strips to overlay an outer edge of the respective top sheet strip.

15. A method according to any one of Claims 10 to 14 further including operating a belly elastic infeed (80) to insert belly elastic strands under tension in the feed direction between the leg section of the first back sheet strip and the third top sheet strip; and
securing the belly elastic strands to the first back sheet strip by means of an adhesive applicator (76).

16. A method according to any one of Claims 10 to 15 further including operating at least one spreading unit (62,64) to space the first and second back sheet strips from each other in a direction transverse to the feed direction and to locate the leg section of the first back sheet strip between the waist section of the first back sheet strip and the waist section of the second back sheet strip.

17. A method according to any one of Claims 10 to 16 further including the step of feeding the first and second continuous webs from a single roll of material arranged to deliver a single feed web in the feed direction and operating the at least one slitting unit and the at least one spreading unit to slit along a fourth cut line extending in the feed direction to form the first and second continuous webs or to slit along the first, second and third cut lines to form the first and second back sheet strips and the first, second, and third top sheet strips.
